# EUROPEAN PATENT SPECIFICATION

(11) **EP 1 839 662 B1**
(45) Date of publication and mention of the grant of the patent: **24.03.2010**
(21) Application number: 06711726.7
(22) Date of filing: 16.01.2006
(51) Int. Cl.: A61K 31/551, A61K 45/00, A61P 35/00, A61P 35/02, C07D 243/12

(54) **ANTITUMOR AGENT**
KREBSMITTEL
AGENT ANTITUMORAL

(30) Priority: 19.01.2005 JP 2005011158
(43) Date of publication of application: 03.10.2007
(73) Proprietor: ZERIA PHARMACEUTICAL CO., LTD., Tokyo 103-8351 (JP)
(72) Inventor: YOSHINAGA, Koji, shi Kobuna-cho, Chuo-ku, Tokyo, 103-8351 (JP); KAWASAKI, Daisuke, shi Kobuna-cho, Chuo-ku, Tokyo, 103-8351 (JP); EMORI, Yutaka, shi Kobuna-cho, Chuo-ku, Tokyo, 103-8351 (JP)
(74) Representative: Hartz, Nikolai
(86) International application number: PCT/JP2006/300445
(87) International publication number: WO 2006/077793

(56) References cited:
- EP-A- 0 945 445
- EP-A- 1 234 818
- EP-A- 1 391 203
- WO-A1-01/40197
- WO-A1-99/64403
- WO-A1-02/092096
- JP-A- 06 080 650
- JP-A- 08 040 908
- WATSON S A; STEELE R J C: "Gastrin antagonists in the treatment of gastric cancer" ANTI-CANCER DRUGS, vol. 4, no. 6, 1 January 1993 (1993-01-01), pages 599-604, XP009061993
- TAKHAR A S; EREMIN O; WATSON S A: "The role of gastrin in colorectal carcinogenesis" SURGEON, vol. 2, no. 5, 1 October 2004 (2004-10-01), pages 251-257, XP009061845

## Description

### Technical Field

The present invention relates to an antitumor agent, and more particularly to an antitumor agent useful for the treatment or prevention of gastrointestinal cancer.

### Background Art

In Japan, mortality rate from cancer has been increasing, and since 1981 cancer has been Japan's leading cause of death. In 2002, death toll from cancer was 304,286 (i.e., 241.5 per 100,000), accounting for 31.0% of all deaths. Particularly, the incidence of gastrointestinal cancers such as pancreatic cancer, colon cancer, and gastric cancer is high.
Among these gastrointestinal cancers, pancreatic cancer is known as an intractable cancer. In Japan, only gemcitabine hydrochloride is approved as a chemotherapeutic agent for pancreatic cancer.
However, a chemotherapeutic agent such as gemcitabine hydrochloride or fluorouracil often causes serious side effects (e.g., myelosuppression and interstitial pneumonia), and therefore a limitation is imposed on the interval or period of administration of such a chemotherapeutic agent.
In addition, a limitation is imposed on the dosage form of such a chemotherapeutic agent, since the agent is generally provided in the form of intravenous drip infusion. Therefore, demand has arisen for development of an antitumor agent which replaces such a chemotherapeutic agent.

A chemotherapeutic agent exhibiting cytotoxic or cytocidal effect is generally employed as an antitumor agent, and multi-drug combination chemotherapy is often employed, in view that employment of several chemotherapeutic agents in combination mitigates adverse side effects of the agents and enhances the antitumor effect of the agents. Multi-drug combination chemotherapy, which generally employs in combination pharmaceutical agents exhibiting different mechanisms of action and different side effects, causes a problem in that when a toxicity common to the pharmaceutical agents (e.g., myelosuppression) occurs, the amounts of the respective pharmaceutical agents must be reduced (Non-Patent Document 1). Also, multi-drug combination chemotherapy causes a problem in that a pharmaceutical agent must be replaced by another pharmaceutical agent due to pharmaceutical agent tolerance.
In recent years, mechanisms of, for example, growth, metastasis, invasion, and malignant progression of cancer have been elucidated at the molecular level, and several target based pharmaceutical agents targeting specific molecules have been developed. Such a molecular target pharmaceutical agent generally exhibits low cytotoxicity, and is envisaged to exhibit reduced side effects, as compared with a conventional chemotherapeutic agent exhibiting cytotoxic effect. Such a target based pharmaceutical agent, which exhibits its effects when employed singly, has also become of interest as a pharmaceutical agent used in combination with a chemotherapeutic agent (Non-Patent Document 2).
Previously, cancer treatment had been evaluated solely on the basis of shrinkage of cancer due to the cytotoxic effect of a chemotherapeutic agent employed. However, in recent years, improvement in quality of life (QOL), suppression of metastasis, or prolongation of survival time has been considered useful evaluation items of evaluating cancer treatment, and employment of a chemotherapeutic agent and a target based pharmaceutical agent in combination is considered a promising cancer treatment (Non-Patent Document 3)

Gastrin is a gastrointestinal hormone which is considered a growth factor of tumor cells. As has been revealed, a gastrin receptor gene is expressed in cells of pancreatic cancer, colon cancer, or gastric cancer (i.e., a gastrointestinal cancer), whereby a potent cell growth property is exhibited in response to gastrin (Non-Patent Documents 4 and 5).
As has been reported, similar to the case of such a gastrointestinal cancer, a gastrin receptor gene is expressed in the case of leukemia, pituitary tumor, small cell lung cancer, thyroid cancer, or neuroastrocytoma, and gastrin can function as a cancer cell growth factor (Non-Patent Document 6).

Previously, an increase in cell growth had been considered to occur mainly through a pathway in which gastrin stimulates a gastrin receptor present on the surface of cells. However, recent studies suggest that there exists a pathway for increasing cell growth by gastrin in which gastrin is bound to a gastrin receptor, and then is taken into cells through endocytosis (Non-Patent Document 7); and that there exists another pathway in which gastrin is bound to a gastrin-binding protein present in cells, thereby regulating cell growth (Non-Patent Documents 8 and 9).
As has also been reported, glycine-extended gastrin, which is a precursor of gastrin, is bound to a non-identified receptor in addition to a gastrin receptor, thereby regulating cell growth (Non-Patent Documents 10 and 11). Therefore, gastrin-mediated cell growth is considered to occur through a plurality of pathways.

Conventionally developed gastrin receptor antagonists are compounds targeting only gastrin receptors, and thus such a conventional gastrin receptor antagonist does not exhibit a consistent and reliable antitumor effect. For example, it has been reported that L-365,260, which is a benzodiazepine compound, suppresses gastrin-induced tumor growth in a human pancreatic cancer PANC-1 xenograft mouse model, but does not suppress tumor growth without stimulation by gastrin (Non-Patent Document 12). Similar results have been reported in the case of CR2093, which is a glutamic acid derivative (Non-Patent Document 13).

These data indicate that a gastrin receptor antagonist suppresses only cancer cell growth induced by forced external gastrin stimulation; i.e., cancer cell growth induced by non-physiological gastrin stimulation. Therefore, a gastrin receptor antagonist, which loses cell growth suppressive effect under physiological conditions, is considered to exhibit insufficient effect as an antitumor agent.

CI-988, which is a C-terminal pentapeptide derivative of CCK, is known as a potent gastrin receptor antagonist. However, as has been reported, when orally administered to a human colon cancer xenograft mouse at a dose of 50 mg/kg, CI-988 exhibits no cell growth suppressive effect, although when orally administered at a dose of 25 mg/kg, CI-988 exhibits cell growth suppressive effect without non-physiological gastrin stimulation (Non-Patent Document 14).

YF476, which is a benzodiazepine compound, is known as a selective and potent gastrin receptor antagonist. Patent Document 1 discloses that YF476 exhibits tumor-shrinking effect in a pancreatic cancer or colon cancer xenograft model. However, the patent document describes that this effect is only observed in the case where YF476 is administered at a high dose of 200 mg/kg or more, and that it is not clear whether or not the mechanism of action of YF476 is mediated by a gastrin receptor.

As described above, numerous gastrin receptor antagonists have been developed, but no established conclusion has been obtained regarding the antitumor effect of such an antagonist. Specifically, it has not been described that gastrin receptor antagonistic effect has a simple correlation with antitumor effect, and the role that a gastrin receptor plays in cancer has not yet been fully elucidated.
Meanwhile, it is not actually clear whether or not a 1,5-benzodiazepine compound described in Patent Document 2 and having gastrin antagonistic effect exhibits useful antitumor effect.
Patent Document 1: WO 02/092096
Patent Document 2: WO 01/40197
Non-Patent Document 1: Nippon Rinsho 2003, 61, 6, 1015-1020
Non-Patent Document 2: Nippon Rinsho 2004, 62, 7, 1232-1240
Non-Patent Document 3: J Clin Oncol 2003, 21, 7, 1404-1411
Non-Patent Document 4: Am J Physiol 1985, 249, G761-769
Non-Patent Document 5: Am J Physiol 1994, 266, R277-283
Non-Patent Document 6: Igaku no Ayumi 1998, 184, 4, 260-261
Non-Patent Document 7: Cell Tissue Res. 1997, 287, 325-333
Non-Patent Document 8: J Gastroenterol Hepatol. 1995, 10, 215-232
Non-Patent Document 9: Eur J Pharmacol. 2000, 388, 9-15
Non-Patent Document 10: Science 1994, 265, 410-412
Non-Patent Document 11: Regul Pept. 2000, 93, 37-44
Non-Patent Document 12: Am J Physiol. 1995, 268, R135-141
Non-Patent Document 13: Br J Cancer. 1992, 65, 879-883
Non-Patent Document 14: Clin Exp Pharmacol Physiol. 1996, 23, 438-440

### Disclosure of the Invention

### Problems to be Solved by the Invention

An object of the present invention is to provide an antitumor agent; in particular, an antitumor agent useful for the treatment and/or prevention of, for example, gastrointestinal cancer, leukemia, pituitary tumor, small cell lung cancer, thyroid cancer, and neuroastrocytoma.

### Means for Solving the Problems

The present inventors have conducted extensive studies on the antitumor effect of a 1,5-benzodiazepine derivative described in WO 01/40197 or a pharmaceutically acceptable salt thereof, and as a result have found that the compound exhibits good antitumor effect.

Accordingly, the present invention provides an antitumor agent containing, as an active ingredient, a 1,5-benzodiazepine derivative which is (R)-(-)-3-[3-(1-tert-butyl carbonyl methyl-2-oxo-5-cyclohexyl-1,3,4,5-tetrahydro-2H-1,5-benzodiazepin-3-yl)ureido]benzoic acid or a pharmaceutically acceptable salt thereof (compound A) for use in the prevention or treatment of a gastrointestinal cancer.

The present invention also provides use of a 1,5-benzodiazepine derivative represented by compound A for producing an antitumor agent for a gastrointestinal cancer.
The present invention also provides a method for treating a gastrointestinal cancer, which includes administering, in an effective amount, a 1,5-benzodiazepine derivative represented by compound A.

### Effects of the Invention

The compound according to the present invention exhibits no such cytocidal effect that a conventional chemotherapeutic agent has exhibited, and does not exhibit serious side effects in safety tests using animals; i.e., the compound has low risk of serious side effects (e.g., myelosuppression and interstitial pneumonia), which would otherwise be caused by a conventional chemotherapeutic agent. Therefore, the compound is useful as an antitumor pharmaceutical agent for, for example, gastrointestinal cancer, leukemia, pituitary tumor, small cell lung cancer, thyroid cancer, and neuroastrocytoma.
Since the pharmaceutical agent according to the present invention exhibits low toxicity, the pharmaceutical agent can be administered in a continuous manner, and can be orally administered. Therefore, the pharmaceutical agent can be prepared in a simple dosage form, as compared with the case of a conventional chemotherapeutic agent.
When the pharmaceutical agent according to the present invention is employed in multi-drug combination chemotherapy, the dose of an antitumor pharmaceutical agent exhibiting severe side effects can be reduced, probably realizing multi-drug combination chemotherapy exhibiting good antitumor effect and reduced side effects. When the pharmaceutical agent is administered in a continuous manner even after administration of a conventional chemotherapeutic agent, the pharmaceutical agent is envisaged to exhibit the effect of suppressing tumor growth; i.e., the pharmaceutical agent can also be employed as a tumor-preventive agent for a gastrointestinal cancer

### Best Modes for Carrying Out the Invention

Among compounds of the invention are (R)-(-)-3-[3-(1-tert-butylcarbonylmethyl-2-oxo-5-cyclohexyl-1,3,4,5-tetrahydro-2H-1,5-benzodiazepin-3-yl)ureido]benzoic acid or a pharmaceutically acceptable salt thereof (compound A).

Examples of salts of compound A include inorganic salts such as a sodium salt, a potassium salt, a calcium salt, and a magnesium salt; organic salts such as an ammonium salt, a pyridine salt, a triethylamine salt, an ethanolamine salt, an (R)- or (S)-form α-phenethylamine salt, a benzylamine salt, and a 4-methylbenzylamine salt; and acid addition salts with organic and inorganic acids. Of these, basic salts are preferred. Among basic salts, inorganic salts are more preferred. Among inorganic salts, alkaline earth metal salts are preferred, with a calcium salt being particularly preferred.
As used herein, "compound A" encompasses its optically active isomers, diastereomers, solvates (e.g., hydrates), and crystal polymorphs.

Compound A can be produced through the method described in WO 01/40197.

As described below in Examples, compound A suppresses growth of various gastrointestinal cancers, and statistically significantly prolongs the survival time of a cancer-bearing host. Therefore, compound A is useful as a pharmaceutical agent for the prevention or treatment of various tumors. When compound A was administered to rats and dogs at a dose of 1,000 mg/kg for 28 consecutive days, no deaths were observed. In addition, no abnormality was found in body weight, feed intake, ophthalmological test, urine test, organ weight, autopsy finding, and histopathological test; i.e., compound A exhibits very high safety.

No particular limitation is imposed on the gastrointestinal cancer to which the antitumor agent according to the present invention is applied.
The antitumor agent according to the present invention is useful for the prevention and/or treatment of gastrointestinal cancer (in particular, pancreatic cancer, colon cancer, or gastric cancer).

The antitumor agent according to the present invention may contain a pharmaceutically acceptable carrier or adjuvant, and may be administered orally or parenterally. The antitumor agent may be administered orally in the form of a solid product such as a tablet, a granule, a powder, or a capsule. For the preparation of such a solid product, the antitumor agent may be combined with an appropriate additive, such as an excipient (e.g., lactose, mannitol, cornstarch, or crystalline cellulose), a binder (e.g., a cellulose derivative, gum arabic, or gelatin), a disintegrant (e.g., carboxymethylcellulose calcium), or a lubricant (e.g., talc or magnesium stearate).
Such a solid product may be prepared into a controlled-release product by use of a coating base material such as hydroxymethylcellulose phthalate, hydroxypropylmethylcellulose acetate succinate, cellulose acetate phthalate, or methacrylate copolymer. The antitumor agent may also be prepared into a liquid product such as a solution, a suspension, or an emulsion.

The antitumor agent according to the present invention may be administered parenterally in the form of an injection. For the preparation of an injection, the antitumor agent may be combined with, for example, water, ethanol, glycerin, or a conventionally employed surfactant. The antitumor agent may also be prepared into a suppository by use of an appropriate base material.

The dose of compound A contained in the antitumor agent according to the present invention is appropriately determined in consideration of the administration method and product form thereof, as well as the symptom, age, sex, etc. of individual patients in need thereof. The daily oral dose of compound (1) for an adult is typically 10 to 1,000 mg, preferably 50 to 600 mg, more preferably 180 to 500 mg. Preferably, the daily oral dose is administered once a day, or in a divided manner (twice to three times a day).

The antitumor agent according to the present invention may be administered in combination with an antitumor agent employed in multi-drug combination therapy (i.e., at least one antitumor agent other than the antitumor agent according to the present invention) or with radiation therapy, in which these antitumor agents may be administered simultaneously or separately at the same frequency of dosage or different frequencies through the same administration method or different administration methods. Thus, the antitumor agent according to the present invention may be employed in combination with multi-drug combination therapy or with radiation therapy for treating cancer patients.

When the antitumor agent according to the present invention is employed in multi-drug combination therapy, the antitumor agent may be added to various pharmaceutical agents employed in the combination therapy, or may be substituted for one to two anticancer agents among the pharmaceutical agents. Examples of antitumor agents which are preferably employed in combination with the antitumor agent according to the present invention include, but are not limited to, antimetabolites such as fluorouracil, gemcitabine hydrochloride, methotrexate, cytarabine, and fludarabine; antitumor antibiotics such as bleomycin hydrochloride, mitomycin C, doxorubicin hydrochloride, daunorubicin hydrochloride, and idarubicin hydrochloride; alkylating agents such as busulfan, coordination metal complexes (carboplatin and cisplatin), cyclophosphamide, dacarbazine, and melphalan; nonsteroidal aromatase inhibitors such as anastrozole and exemestane; immunotherapeutic agents such as trastuzumab and rituximab; mitotic inhibitors such as paclitaxel, docetaxel hydrate, vincristine sulfate, and vinblastine sulfate; topoisomerase inhibitors such as irinotecan hydrochloride; hormone therapy agents such as tamoxifen citrate and leuprorelin acetate; and other antitumor agents such as calcium levofolinate, tyrosine kinase inhibitors (e.g., gefitinib), monoclonal antibodies (e.g., cetuximab and bevacizumab), matrix metalloprotease inhibitors, and farnesyltransferase inhibitors. Particularly preferably, the antitumor agent according to the present invention is added during use of gemcitabine hydrochloride, which is known to exhibit the effect of treating pancreatic cancer, or the antitumor agent is added to combination therapy employing gemcitabine hydrochloride and another chemotherapeutic agent (e.g., fluorouracil, calcium levofolinate, irinotecan hydrochloride, or a coordination metal complex).

When compound A is employed in combination with other antitumor agents, the dose of compound A or the antitumor agents is appropriately determined in consideration of, for example, the identity of each of the antitumor agents, the symptom of a patient in need thereof, and the administration method thereof. In multi-drug combination therapy, the dose of compound A is similar to that described above. The administration period, administration frequency, and dosage form of compound A are optimized in consideration of the identity of each of the antitumor agents employed in combination with compound A. Specifically, compound A and at least one antitumor agent (preferably, one to four antitumor agents) are administered simultaneously or separately at the same frequency or different frequencies in the same dosage form or different dosage forms. In multi-drug combination therapy, preferably, compound A is orally or intravenously administered one or more times a day. An antitumor agent is generally administered through intravenous infusion, but is more preferably administered by an oral route in view that a simple dosage form can be selected.

As described below in Examples, when the antitumor agent according to the present invention is employed in combination with another antitumor agent, excellent antitumor effect is attained without an increase in side effects. Therefore, when the pharmaceutical agent according to the present invention is employed in multi-drug combination chemotherapy, the dose of another antitumor agent exhibiting severe side effects can be reduced. The antitumor agent according to the present invention can be continuously administered even after the chemotherapy, and thus further excellent antitumor effect is highly envisaged to be obtained.

It has been reported that compound A has a high binding affinity to a rat gastrin receptor (Ki value = 0.24 nM), and intraduodenal administration of compound A at doses of 0.17 mg/kg suppresses gastrin-stimulated gastric acid secretion in rat by 50% (Gastroenterology 2001; A-311: 1605). In contrast, the antitumor agent for present invention required more high doses for expression of antitumor effect.

### Examples

The present invention will next be described in detail with reference to Examples and Comparative Examples, but the invention is not limited to these Examples. Antitumor effect and toxicity of compound A will be described in Examples 1 to 6. The preparation of the antitumor agent for the present invention will be described in Formulation Examples 1 to 3.

### Example 1

3 x 10⁶ cells of human pancreatic cancer cells (MIAPaCa 2) were subcutaneously implanted into right abdomen of female Balb/c nude mice. After the tumor volume had become 100 mm³ or more, calcium (R)-(-)-3-[3-(1-tert-butylcarbonylmethyl-2-oxo-5-cyclohexyl-1,3,4,5-tetrahydro-2H-1,5-benzodiazepin-3-yl)ureido]benzoate (hereinafter called "compound A1") was orally administered to mice in administration groups at doses of 10, 30, and 100 mg/kg once daily for 21 days. On the day following the final administration, the tumor was removed and weighed. For comparison, vehicle was orally administered to mice in a control group, and the tumor weight was measured in similar manner to that described above. Percent inhibition of tumor growth was calculated based on the tumor weights in each administration group vursus that in a control group. As a result, percent inhibition of 30 mg/kg and 100 mg/kg of compound A1 were 40% and 42%, respectively. The administration of compound A1 significantly inhibited MIAPaCa 2 tumor growth in a dose-dependent manner.

### Example 2

1 ×10⁶ cells of human pancreatic cancer cells (PAN1VC) were implanted into the pancreas of male nude mice. From the day following tumor implantation, compound A1 was orally administered at doses of 30 mg/kg and 100 mg/kg once daily for 36 days. One, three, and six days after tumor implantation, gemcitabine hydrochloride (Gemzar Injection(R)) was intravenously administered at a dose of 5 mg/kg. Percent inhibition of tumor growth was calculated based on the tumor weights in each administration groups versus that in a control group. As a result, percent inhibition of single dose of gemcitabine hydrochloride (Gemzar Injection(R), 30 mg/kg of compound A1, and 100 mg/kg of compound A1 were 32%, 19%, and 23%, respectively. In contrast, when gemcitabine hydrochloride (Gemzar Injection(R)) and compound A1 were administered in combination, percent inhibition of 30 mg/kg and 100 mg/kg of compound A1 were 73% and 84%, respectively. These data indicate that the combination of compound A1 and gemcitabine hydrochloride (Gemzar Injection(R)) exhibits excellent antitumor effect.

### Example 3

1.5 × 10⁶ cells of human colon cancer cells (C170HM2) were intraperitoneally injected into male nude mice. After implantation, in administration groups, compound A1 was orally administered to mice at doses of 3 mg/kg and 30 mg/kg once daily. Meanwhile, in a positive control group, the combination of 5-fluorouracil (hereinafter called "5-FU") and leucovorin were intravenously administered (for each compound, 25 mg/kg/injection) one, four, seven, and 10 days after tumor implantation. Forty days after implantation of C170HM2 tumor, the weight of tumor-metastasized liver was measured. Administration of compound A1 at doses of 3 mg/kg and 30 mg/kg resulted in the inhibition of tumor metastasis to the liver by 73% and 81%, respectively.
In contrast, percent inhibition of metastasis in a positive control group was 63%. These data indicate that compound A1 exhibits antimetastatic effect comparable to or greater than that of a chemotherapeutic agent.

### Example 4

5 × 10⁵ cells of human gastric cancer cells (MGLVA1) were intraperitoneally injected into female SCID mice. After implantation, compound A1 was orally administered to mice at doses of 3 mg/kg and 30 mg/kg once daily. The prolongation of survival time by compound A1 was evaluated because this model using MGLVA1 was lethal model. On day 6 after the administration start, the survival rate was 6.7% in a control group, whereas the survival rate was 46.7% in a group of administration of compound A1 at a dose of 30 mg/kg. These data indicate that compound A1 exhibits the effect of prolonging survival time after tumor implantation.

### Example 5

1 × 10⁶ cells of human colon cancer cells (HT-29) were subcutaneously implanted into right abdomen of female Balb/c nude mice. From four days after tumor implantation, compound A1 was orally administered to mice in administration groups at doses of 10, 30, and 100 mg/kg once daily for 17 days. On the day following the final administration, the tumor was removed and weighed. In a control group, vehicle was orally administered to mice, and the tumor weight was measured in similar manner to that described above. Percent inhibition of tumor growth was calculated based on the tumor weights in each administration groups versus that in a control group.
As a result, percent inhibition of 30 mg/kg and 100 mg/kg of compound A1 were 44% and 50%, respectively. The administration of compound A1 significantly inhibited tumor growth in a dose-dependent manner.

### Example 6

1 × 10⁶ cells of human colon cancer cells (HT-29) were subcutaneously implanted into right abdomen of female Balb/c nude mice. From 10 days after tumor implantation, compound A1 was orally administered to mice in an administration group at a dose of 30 mg/kg once daily for 12 days.
For comparison, 5-FU was intraperitoneally administered to mice in positive control groups at doses of 3, 10, and 30 mg/kg once daily for 12 days.
In addition, the combination of compound A1 (30 mg/kg) and 5-FU (3, 10, or 30 mg/kg) were administered to mice in each combination groups. On the day following the final administration, the tumor was removed and weighed. In a control group, vehicle was administered to mice, and the tumor weight was measured in similar manner to that described above. Percent inhibition of tumor growth was calculated based on the tumor weights in each administration groups versus that in a control group. The percent inhibition of single administration of compound A1 at a dose of 30 mg/kg was 34%. The percent inhibition of single administration of 5-FU at doses of 3, 10, and 30 mg/kg were 24%, 30%, and 58%, respectively.
In contrast, when compound A1 at a dose of 30 mg/kg and 5-FU at a dose of 3, 10, or 30 mg/kg were administered in combination, percent inhibition was 31%, 54%, or 76%, respectively. These data indicate that the combination of compound A1 and 5-FU exhibits excellent antitumor effect.

### Example 7

A small piece (70 to 80 mg) of human pancreatic cancer cells (PANC-1) was implanted into the pancreas of female SCID mice (15 mice for each group). In a group of mice, from seven days after implantation, compound A1 was orally administered at a dose of 100 mg/kg once daily. In another group of mice, on seven, 10, and 14 days after the implantation, gemcitabine hydrochloride (Gemzar Injection(R)) as a positive control was intravenously injected at a dose of 100 mg/kg. The prolongation of survival time by compound A1 was evaluated because this model using PANC-1 was lethal model. On forty days after the administration start (on 46 days after the implantation), the survival rate in a control (vehicle administration) group was 46.7%, whereas the survival rate in the compound A1 administration (100 mg/kg) group was 86.7%. Meanwhile, the survival rate in the gemcitabine hydrochloride administration group was 93.3%. These data indicate that compound A1 exerts the survival benefit after tumor implantation comparable to a chemotherapeutic agent.

### Example 8

A small piece (70 to 80 mg) of human pancreatic cancer cells (PANC-1) was implanted into the pancreas of female SCID mice (15 mice for each group). In a group of mice, from seven days after the implantation, compound A1 was orally administered at a dose of 100 mg/kg once daily. In another group of mice, on seven, 10, and 14 days after implantation, gemcitabine hydrochloride (Gemzar Injection(R)) was intravenously administered at a dose of 100 mg/kg. The prolongation of survival time by compound A1 was evaluated because this model using PANC-1 was lethal model. As shown in Table 1, administration of gemcitabine hydrochloride ("GEM" in Table 1) (100 mg/kg) and compound A1 (100 mg/kg) in combination prolongs survival time. These data indicate that administration of compound A1 and a chemotherapeutic agent in combination exhibits the survival benefit after tumor implantation.

**[Table 1]**

| | Control | Compound A1 | GEM (100) × 3 | GEM (100) × 3 + compound A1 (100) |
|---|---|---|---|---|
| Days of death of the last individual | 56 | 61 | 54 | 63* |
| Average survival time (days) | 43 | 49.5 | 46.9 | 50.9 |
| Median survival time (days) | 39 | 51 | 48 | 54 |
| Survival rate (%) at 50 days after initiation of administration (56 days after implantation) | 40 | 53.3 | 20 | 66.7 |
| Survival rate (%) at 60 days after initiation of administration (66 days after implantation) | 0 | 6.7 | 0 | 20* |

| | | | | |
|---|---|---|---|---|
| * < 0.05 compared to control (by Kaplan-Meier method, multiple logrank tests) | | | | |

### Test Example 1 Toxicity test through 28-day repeated oral administration to rats

Compound A1 was orally administered to six-week-old male and female SD rats at a dose of 30, 100, 300, or 1,000 mg/kg for 28 days in a repeated manner. In any group, no deaths were observed, and no abnormality was found in body weight, feed intake, ophthalmological test, urine test, organ weight, autopsy finding, and histopathological test.

### Test Example 2 Toxicity test through 28-day repeated oral administration to dogs

Compound A1 was orally administered to eight-month-old male and female beagle dogs at a dose of 30, 100, 300, or 1,000 mg/kg for 28 days in a repeated manner. In any group, no deaths were observed, and no abnormality was found in body weight, feed intake, ophthalmological test, electrocardiogram, blood pressure, urine test, hematological test, blood biochemical test, organ weight, and autopsy finding.

### Formulation Example 1

Compound A1 (20 g), lactose (315 g), cornstarch (125 g), and crystalline cellulose (25 g) are uniformly mixed together, and 7.5% aqueous hydroxypropylcellulose solution (200 mL) is added to the resultant mixture. The mixture is granulated by means of an extrusion granulator employing a screen (mesh diameter: 0.5 mm), and immediately thereafter, the resultant product is formed into spherical shape by means of a marumerizer, followed by drying, to yield granules.

### Formulation Example 2

Compound A1 (20 g), lactose (100 g), cornstarch (36 g), crystalline cellulose (30 g), carboxymethylcellulose calcium (10 g), and magnesium stearate (4 g) are uniformly mixed together. The resultant mixture is formed into tablets (200 mg each) by means of a single-punch tableting machine having a pestle of 7.5 mm in diameter.

### Formulation Example 3

Compound A1 (100 mg), sodium acetate (2 mg), acetic acid (for adjusting pH to 5.8) (appropriate amount), and distilled water (balance) (total: 10 mL/vial) are formulated into an injection through a customary method.

## Claims

1. An antitumor agent for use in the treatment and/or prevention of gastrointestinal cancer, comprising as active ingredients:
(A) a 1,5-benzodiazepine derivative which is (R)-(-)-3-[3-(1-*tert-*butylcarbonylmethyl-2-oxo-5-cyclohexyl-1,3,4,5-tetrahydro-2H-1,5-benzodiazepin-3-yl)ureido]benzoic acid or a pharmaceutically acceptable salt thereof; and
(B) another antitumor agent which is an antimetabolite.

2. The antitumor agent according to claim 1, wherein the pharmaceutically acceptable salt of the 1,5-benzodiazepine derivative is a calcium salt.

3. The antitumor agent according to claim 1 or 2 , which is an agent for the treatment and/or prevention of pancreatic cancer.

4. The antitumor agent according to any one of claims 1 to 3, wherein said antitumor agent (B) is gemcitabine.

5. The antitumor agent according to any one of claims 1 to 4, wherein said ingredient (A) is a peroral agent.

6. Use of
(A) a 1,5-benzodiazepine derivative which is (R)-(-)-3-[3-(1-*tert-*butylcarbonylmethyl-2-oxo-5-cyclohexyl-1,3,4,5-tetrahydro-2H-1,5-benzodiazepin-3-yl)ureido]benzoic acid or a pharmaceutically acceptable salt thereof; and
(B) another antitumor agent which is an antimetabolite, for the production of an antitumor agent for the treatment and/or prevention of gastrointestical cancer.

7. The use according to claim 6, wherein the pharmaceutically acceptable salt of the 1,5-benzodiazepine derivative is a calcium salt.

8. The use according to claim 6 or 7 , wherein said antitumor agent is an agent for the treatment and/or prevention of pancreatic cancer.

9. The use according to any one of claims 6 to 8, wherein said antitumor agent (B) is gemcitabine.

10. The use according to any one of claims 6 to 9, wherein said ingredient (A) is a peroral agent.

## Patentansprüche

1. Antitumormittel zur Verwendung bei der Behandlung von und/oder Vorbeugung gegen Krebs des Magendarmtrakts, umfassend als aktive Bestandsteile:
(A) ein 1,5-Benzodiazepinderivat, das ein (R)-(-)-3-[3-(1-tert-Butylcarbonylmethyl-2-oxo-5-cyclohexyl-1,3,4,5-tetrahydro-2H-1,5-benzodiazepin-3-yl)ureido]benzoesäure oder ein pharmazeutisch unbedenkliches Salz davon ist; und
(B) ein weiteres Antitumormittel, das ein Antimetabolit ist.

2. Das Antitumormittel nach Anspruch 1, wobei das pharmazeutisch unbedenkliche Salz des 1,5-Benzodiazepinderivats ein Kalziumsalz ist.

3. Das Antitumormittel nach Anspruch 1 oder 2, das ein Mittel zur Behandlung von und/oder Vorbeugung gegen Magenkrebs ist.

4. Das Antitumormittel nach einem der Ansprüche 1 bis 3, wobei das Antitumormittel (B) Gemcitabine ist.

5. Das Antitumormittel nach einem der Ansprüche 1 bis 4, wobei der Bestandteil (A) ein peroral verabreichtes Mittel ist.

6. Verwendung von
(A) einem 1,5-Benzodiazepinderivat, das (R)-(-)-3-[3-(1-tert-Butylcarbonylmethyl-2-oxo-5-cyclohexyl-1,3,4, 5-tetrahydro-2H-1,5-benzodiazepin-3-yl)ureido]benzoesäure oder ein pharmazeutisch unbedenkliches Salz davon ist; und
(B) einem weiteres Antitumormittel, das ein Antimetabolit ist, zur Herstellung eines Antitumormittels zur Behandlung von und/oder Vorbeugung gegen Krebs des Magendarmtrakts.

7. Die Verwendung nach Anspruch 6, wobei das pharmazeutisch unbedenkliche Salz des 1,5-Benzodiazepinderivats ein Kalziumsalz ist.

8. Die Verwendung nach Anspruch 6 oder 7, wobei das Antitumormittel ein Mittel zur Behandlung von und/oder Vorbeugung gegen Magenkrebs ist.

9. Die Verwendung nach einem der Ansprüche 6 bis 8, wobei das Antitumormittel (B) Gemcitabine ist.

10. Die Verwendung nach einem der Ansprüche 6 bis 9, wobei der Bestandteil (A) ein peroral zu verabreichendes Mittel ist.

## Revendications

1. Agent antitumoral pour utilisation dans le traitement et/ou la prévention d'un cancer gastrointestinal, comprenant, à titre d'ingrédients actifs :
(A) un dérivé de 1,5-benzodiazépine qui est l'acide (R)-(-)-3-[3-(1-tert-butylcarbonylméthyl-2-oxo-5-cyclohexyl-1,3,4,5-tétrahydr.o-2H-1,5-benzodiazépin-3-yl)-uréido]benzoïque ou un sel pharmaceutiquement acceptable de celui-ci ; et
(B) un autre agent antitumoral qui est un antimétabolite.

2. Agent antitumoral selon la revendication 1, dans lequel le sel pharmaceutiquement acceptable du dérivé de 1,5-benzodiazépine est un sel de calcium.

3. Agent antitumoral selon la revendication 1 ou 2, qui est un agent pour le traitement et/ou la prévention d'un cancer du pancréas.

4. Agent antitumoral selon l'une quelconque des revendications 1 à 3, dans lequel ledit agent antitumoral (B) est la gemcitabine.

5. Agent antitumoral selon l'une quelconque des revendications 1 à 4, dans lequel ledit ingrédient (A) est un agent per-oral.

6. Utilisation
(A) d'un dérivé de 1,5-benzodiazépine qui est l'acide (R)-(-)-3-[3-(1-tert-butylcarbonylméthyl-2-oxo-5-cyclo-hexyl-1,3,4,5-tétrahydro-2H-1,5-benzodiazépin-3-yl)-uréido]benzoïque ou un sel pharmaceutiquement acceptable de celui-ci ; et
(B) d'un autre agent antitumoral qui est un antimétabolite,
pour la production d'un agent antitumoral pour le traitement et/ou la prévention d'un cancer gastrointestinal.

7. Utilisation selon la revendication 6, dans laquelle le sel pharmaceutiquement acceptable du dérivé de 1,5-benzodiazépine est un sel de calcium.

8. Utilisation selon la revendication 6 ou 7, dans laquelle ledit agent antitumoral est un agent pour le traitement et/ou la prévention d'un cancer du pancréas.

9. Utilisation selon l'une quelconque des revendications 6 à 8, dans laquelle ledit agent antitumoral (B) est la gemcitabine.

10. Utilisation selon l'une quelconque des revendications 6 à 9, dans laquelle ledit ingrédient (A) est un agent per-oral.
